# FASCICULE DE BREVET EUROPEEN

(11) **EP 3 920 978 B1**
(45) Date de publication et mention de la délivrance du brevet: **07.06.2023**
(21) Numéro de dépôt: 20713942.9
(22) Date de dépôt: 07.02.2020
(51) Int. Cl.: A61L 2/18, A61L 2/24, B08B 1/00, B08B 9/02, B62B 5/06, E05B 1/00, E05B 5/00

(54) **SYSTÈME DE NETTOYAGE ET/OU DE DÉSINFECTION D'UN TUBE CREUX, NOTAMMENT UNE BÉQUILLE DE POIGNÉE DE PORTE**
SYSTEM ZUR REINIGUNG UND/ODER DESINFEKTION EINES HOHLROHRES, INSBESONDERE EINES TÜRGRIFFHEBELS
SYSTEM FOR CLEANING AND/OR DISINFECTING A HOLLOW TUBE, IN PARTICULAR A DOOR HANDLE LEVER

(30) Priorité: 08.02.2019 FR 1901282
(43) Date de publication de la demande: 15.12.2021
(73) Titulaire: Céclean, 60200 Compiegne (FR)
(72) Inventeur: CHAUSSARD, Philippe, 60200 Compiegne (FR); VAUQUELIN, Aurélien, 60200 Compiegne (FR)
(74) Mandataire: Touroude & Associates
(86) Numéro de dépôt international: PCT/FR2020/000026
(87) Numéro de publication internationale: WO 2020/161402

(56) Documents cités:
- DE-B3-102008 047 556
- JP-A- H04 271 884

## Description

### Domaine technique

La présente invention concerne un système automatisé de nettoyage et/ou de désinfection d'un tube creux, pouvant notamment être une béquille de poignée de porte, ou encore un tube d'une barre équipant un moyen de transport, d'une poignée de type bâton de maréchal ou d'une rampe telle que par exemple une rampe d'escalier. L'invention est notamment applicable, sans que cela ne soit exclusif ni limitatif, au domaine des systèmes de nettoyage de poignée de porte dégraissants et bactéricides à l'usage notamment des établissements recevant du public.

L'invention concerne également un ensemble comportant un tel système et au moins une poignée de porte munie d'une béquille de manoeuvre de la poignée et d'un support de la béquille.

### Etat de la technique

Dans les établissements recevant du public, il existe un besoin de pouvoir garantir que l'ouverture de toute porte de l'établissement puisse s'effectuer au travers d'une poignée assurant une absence de germes microbiens et de matières organiques sur celle-ci. En effet, le contact d'une main avec la béquille de manoeuvre d'une poignée de porte est de nature à constituer un risque de transmission de germes infectieux ou d'agents pathogènes. Or, ceci est particulièrement critique dans les hôpitaux par exemple, ou encore dans les cabinets de toilettes installés dans des lieux publics.

Plus généralement, il existe un besoin de pouvoir garantir un nettoyage, sans transmission de germes infectieux ou d'agents pathogènes, de tout tube creux de type béquille de poignée de porte, tube d'une barre équipant un moyen de transport de type bus ou métro, poignée de type bâton de maréchal ou rampe telle que par exemple rampe d'escalier.

Une première solution connue consiste à faire déplacer manuellement par un utilisateur, par translation longitudinale sur le tube creux, un dispositif de nettoyage et/ou de désinfection. Toutefois, un tel contact manuel entre la main de l'utilisateur et le dispositif occasionne des transferts de souches bactériennes et/ou microbiennes sur le tube creux, et ce même si le tube a été nettoyé et/ou désinfecté par le dispositif.

Afin de remédier à l'inconvénient précité et d'éliminer totalement les risques de transmission microbienne ou infectieuse, il est connu des systèmes automatisés de nettoyage et/ou de désinfection d'un tube creux, pouvant être par exemple une béquille de poignée de porte. De tels systèmes permettent de dégraisser et/ou de désinfecter la poignée après chaque utilisation de celle-ci. Un système de ce type est par exemple décrit dans le document brevet GB 2 433 878 A. Le système comprend, outre la béquille de manoeuvre, un réservoir d'un liquide désinfectant et dégraissant ; une bague solidarisée au réservoir et montée coulissante sur la béquille ; et un système d'actionnement de la bague. La bague renferme un tampon absorbant en communication fluidique avec le réservoir, lequel tampon est agencé autour de la béquille. La bague, qui comporte des éléments en matériau magnétique, est mise en mouvement le long de la béquille par couplage magnétique avec une bobine, laquelle bobine forme une première partie du système d'actionnement. La bobine est agencée à l'intérieur de la béquille et est elle-même propulsée par de l'air provenant d'un ensemble piston-cylindre, lequel ensemble forme la seconde partie du système d'actionnement. Cet ensemble piston-cylindre est relié mécaniquement à la porte, de sorte que le mouvement de la porte commande l'actionnement de la bague dans un sens ou dans un autre le long de la béquille.

Toutefois, un premier inconvénient du système proposé dans le document brevet GB 2 433 878 A, dont la bague est mue par un champ magnétique, est qu'il génère des forces radiales qui accentuent les frottements de la bague sur la béquille. Ceci a pour conséquence que le nettoyage obtenu par un tel système est difficilement répétable. En outre, du fait que le système est en boucle ouverte, aucun contrôle ou régulation du mouvement n'est effectué(e). Ainsi, la vitesse du mouvement n'est pas contrôlée par le système. Or, du fait des vitesses relativement élevées atteintes par la bobine mue par une pression ou une dépression d'air, ceci a pour conséquence une mauvaise application du liquide par un tampon trop rapide dans son mouvement. Enfin, un tel système est très encombrant, ne permet pas de garantir que le mouvement de la bague a réellement eu lieu sur toute la longueur de la béquille, et oblige un utilisateur à devoir disposer d'une porte spéciale, compatible avec le système.

Le document brevet DE 10 2008 047556 B3 décrit un système de nettoyage et/ou de désinfection d'une béquille de poignée de porte. Le système comprend, outre la béquille, une bague montée coulissante sur la béquille et un dispositif d'actionnement électromécanique de la bague agencé à l'intérieur de la béquille. La bague renferme un tampon absorbant imbibé d'un liquide désinfectant et/ou dégraissant, le tampon absorbant étant agencé autour de la béquille. Le dispositif d'actionnement électromécanique, sous la forme d'un fuseau de filetage couplé à un moteur, comporte un élément d'entrainement en translation longitudinale de la bague, sous la forme d'un écrou à vis agencé sur le fuseau. Une fente longitudinale est ménagée dans la béquille. L'élément d'entrainement en translation longitudinale fait saillie à travers la fente longitudinale et est mécaniquement lié à la bague, pour le déplacement de la bague en translation longitudinale le long de la béquille.

Toutefois, un inconvénient du système proposé dans le document brevet DE 10 2008 047556 B3 est qu'il ne permet pas un contrôle précis de la position de la bague sur le tube creux formé par la béquille. Par conséquent, du fait de ce manque de contrôle et d'identification de la position de la bague par le système, le système ne permet pas de garantir que le mouvement de la bague s'est effectivement effectué sur toute la longueur du tube. Ceci est préjudiciable à l'efficacité du nettoyage et/ou de la désinfection du tube. Un autre inconvénient de ce système est qu'il est relativement fragile. En effet, si un utilisateur du système (tel qu'un enfant par exemple) vient à tirer à tirer sur la bague, le couplage entre la bague et le fuseau de filetage peut altérer la tige ou le moteur si la bague vient à être tirée de façon trop énergique. Enfin, le bruit généré par le fonctionnement du fuseau de filetage rend un tel système peu compatible avec les lieux publics dans lesquels il doit être installé.

### Résumé de l'invention

L'invention a donc pour but de fournir un système automatisé de nettoyage et/ou de désinfection d'un tube creux qui soit fiable, discret et compact, et permettant d'obtenir un nettoyage répétable, sur toute la longueur du tube, sur une distance et dans un temps donné.

Un autre but de l'invention est de fournir un système automatisé de nettoyage et/ou de désinfection d'un tube creux qui permette un contrôle précis de la position de la bague sur le tube creux.

Un autre but de l'invention est de fournir en particulier un système automatisé de nettoyage et/ou de désinfection d'une béquille de poignée ne nécessitant pas de changer ou de modifier la porte, ni la mortaise de la porte.

A cet effet, selon un premier aspect de l'invention, l'invention concerne un système de nettoyage et/ou de désinfection d'un tube creux, comprenant :
- le tube creux ;
- une bague montée coulissante sur le tube creux, la bague renfermant un tampon absorbant imbibé d'un liquide désinfectant et/ou dégraissant, le tampon absorbant étant agencé autour du tube creux ;
- un dispositif d'actionnement électromécanique de la bague agencé à l'intérieur du tube creux, ledit dispositif comportant un élément d'entrainement en translation longitudinale ;
- une fente longitudinale est ménagée dans le tube creux, l'élément d'entrainement en translation longitudinale faisant saillie à travers ladite fente longitudinale et étant mécaniquement lié à la bague, pour le déplacement de la bague en translation longitudinale le long du tube creux ;
dans lequel le dispositif d'actionnement électromécanique est un potentiomètre linéaire motorisé.

Grâce au dispositif d'actionnement électromécanique de la bague agencé à l'intérieur du tube creux, le système de nettoyage et/ou de désinfection selon l'invention est particulièrement compact. En outre, un tel actionnement électromécanique d'un élément, entraînant la bague en translation longitudinale le long du tube, permet d'obtenir un nettoyage fiable et répétable. Le système selon l'invention permet ainsi de garantir que le contact entre la main de l'utilisateur et le tube creux, à tout moment et à n'importe quel endroit de celui-ci, est toujours propre et désinfecté. De plus, grâce au retour potentiométrique obtenu du potentiomètre, le système selon l'invention permet de garantir que le mouvement de la bague, et donc le nettoyage du tube creux, s'est effectivement effectué sur toute la longueur du tube. En effet, un tel potentiomètre linéaire motorisé permet au système d'obtenir une valeur de tension proportionnelle à la position de la bague. Cette position de la bague peut donc être déterminée, à tout instant, de manière plus précise par le système. Ceci permet avantageusement d'améliorer le contrôle de la position de la bague sur le tube par rapport aux dispositifs connus de l'art antérieur, permettant ainsi d'améliorer l'efficacité du nettoyage et/ou de la désinfection, en s'assurant que toutes les parties du tube sont en contact avec le tampon. En outre, l'utilisation d'un tel potentiomètre linéaire motorisé permet d'obtenir un système de nettoyage et/ou de désinfection fiable et discret.

En outre, dans le cas particulier où le tube creux est une béquille de manoeuvre d'une poignée de porte, l'utilisateur du système de nettoyage et/ou de désinfection selon l'invention n'a pas besoin de disposer d'une porte spéciale pour utiliser le système. Pour une telle utilisation, il est uniquement nécessaire de remplacer une poignée de porte conventionnelle par une poignée de porte équipée du système selon l'invention. Il n'est ainsi nullement nécessaire de changer ou de modifier la porte, ni d'agrandir la mortaise de cette dernière, au contraire de certains systèmes automatisés de nettoyage et/ou de désinfection de l'art antérieur. Une serrure à larder conventionnelle est en outre avantageusement compatible avec une poignée équipée du système selon l'invention.

Des formes particulières du système de nettoyage et/ou de désinfection sont définies selon l'invention.

Avantageusement, le dispositif d'actionnement électromécanique comporte en outre un moteur alimenté électriquement et un ensemble poulies/courroies actionné par le moteur, l'élément d'entrainement en translation longitudinale étant relié à l'ensemble poulies/courroies. Un tel ensemble à poulies/courroies permet d'atteindre des vitesses de déplacement élevées, tout en étant réversible mécaniquement et occasionnant un bruit réduit. Cette réversibilité mécanique du système est particulièrement avantageuse dans le cadre de la présente invention, pour des questions de sécurité mais également pour permettre à un utilisateur de pouvoir bouger manuellement la bague, par exemple pour la maintenance.

Avantageusement, l'élément d'entrainement en translation longitudinale est un chariot mobile monté en translation sur une partie fixe du potentiomètre linéaire motorisé et comportant une languette, et une fente longitudinale est ménagée dans la bague, la languette faisant saillie à travers les fentes longitudinales respectives du tube creux et de la bague.

Avantageusement, le tube creux ou la bague est muni(e) d'au moins un marquage visuel, notamment au moins un marquage coloré ou au moins un repère gravé, ledit au moins un marquage visuel étant indicatif d'une rupture en liquide et/ou en alimentation électrique. Ceci permet d'informer de manière fiable un utilisateur quant à une rupture en liquide et/ou en énergie électrique du système, afin que l'utilisateur puisse ensuite recharger le liquide désinfectant et/ou dégraissant, et/ou remplacer les piles d'alimentation.

Avantageusement, le dispositif d'actionnement électromécanique comporte en outre un microcontrôleur de commande du moteur, relié au moteur. Ceci permet d'assurer une régulation électronique du moteur, qui permet de garantir un comportement répétable et constant de ce dernier, et donc un nettoyage fiable, constant et répétable du tube creux. En outre, une telle régulation électronique garantit une bonne application du liquide sur le tube par le tampon absorbant.

Selon un exemple de réalisation particulier de l'invention, le tube creux est une béquille de manoeuvre d'une poignée de porte.

Avantageusement, le dispositif d'actionnement électromécanique comporte en outre un inclinomètre relié au microcontrôleur, ledit inclinomètre étant configuré pour détecter un mouvement de la béquille et pour transmettre au microcontrôleur un signal de détection correspondant, le microcontrôleur étant configuré pour transmettre au moteur, à l'issue d'une durée prédéfinie écoulée depuis la réception du signal de détection, un signal de commande d'un mouvement de va-et-vient de la bague le long de la béquille. Ceci permet d'obtenir un nettoyage de la béquille fiable et entièrement automatisé, et ce sans gêner l'utilisateur lors de la manipulation de la poignée pour l'ouverture ou la fermeture de la porte. La durée prédéfinie est en effet déterminée au préalable via des tests d'utilisation expérimentaux. Une durée comprise entre une seconde et trois secondes peut par exemple s'avérer adéquate. Selon une caractéristique technique particulière de l'invention, le microcontrôleur comprend des moyens de traitement et des moyens mémoire stockant un code source, le code source comportant des instructions de programme qui, lorsqu'exécutées par les moyens de traitement, déclenchent une séquence de commande du moteur, pour le déplacement de la bague en translation longitudinale le long du tube creux.

Avantageusement, le microcontrôleur comprend en outre un compteur pré-étalonné, le compteur enregistrant un nombre prédéterminé de cycles du système, correspondant à une rupture en liquide et/ou en alimentation électrique, et la séquence de commande du moteur est paramétrée au sein du code source de sorte que, lorsque le compteur a atteint le nombre prédéterminé de cycles, le microcontrôleur commande le moteur pour amener la bague, en fin de mouvement, jusqu'à une position prédéfinie sur le tube creux laissant apparaître le marquage visuel. Le nombre de cycles prédéterminés est par exemple déterminé par des études préalables d'étalonnage, et correspond à une rupture en liquide et/ou en alimentation électrique. Ceci permet d'informer de manière fiable un utilisateur quant à une rupture en liquide et/ou en énergie électrique du système, tout en s'affranchissant de l'utilisation de capteurs et/ou de détecteurs pour déterminer cette rupture.

Selon une caractéristique technique particulière de l'invention, le système comprend en outre un réservoir de liquide désinfectant et/ou dégraissant, la bague étant solidarisée au réservoir, le tampon absorbant étant en communication fluidique avec le réservoir.

Selon une caractéristique technique particulière de l'invention, la bague et le réservoir forment une pièce monobloc. La pièce monobloc est par exemple avantageusement obtenue via un procédé de fabrication par impression en trois dimensions. Ceci permet de fabriquer la bague avec différentes tailles de réservoirs. L'utilisateur final peut ainsi avantageusement choisir le volume de son réservoir. Ce dernier est par exemple établi de base à 10 ml, ce qui permet une autonomie d'environ une semaine dans des conditions standards d'utilisation. En jouant sur le volume du réservoir, l'utilisateur final peut par exemple doubler, voire tripler ce volume.

Selon une variante de réalisation de l'invention, un premier orifice est ménagé dans la bague en regard du tampon absorbant, ledit orifice débouchant dans le réservoir, le réservoir et l'orifice étant configurés pour que du liquide s'écoule depuis le réservoir vers le tampon, à travers l'orifice, lorsque la béquille est inclinée vers le bas. Cette variante de réalisation permet une recharge du tampon par arrosage, du fait de l'effet de gravité, chaque fois que la poignée est actionnée. Cette méthode de recharge du tampon correspond à un cas d'utilisation où la poignée est sollicitée de manière soutenue, par exemple dans une utilisation quotidienne et/ou diurne de la poignée.

Selon une variante ou un complément de réalisation de l'invention, un second orifice est ménagé dans la bague en regard du tampon absorbant, ledit orifice débouchant dans le réservoir, et le système comporte en outre une mèche capillaire agencée à travers l'orifice jusque dans le réservoir, et en contact avec le tampon absorbant, pour permettre un transfert de liquide par capillarité du réservoir vers le tampon absorbant. Cette variante ou ce complément de réalisation permet une recharge du tampon par effet de capillarité. Cette méthode de recharge du tampon correspond à un cas d'utilisation où la poignée est peu sollicitée et/ou le tampon n'est plus alimenté par arrosage, par exemple dans une utilisation peu fréquente et/ou nocturne de la poignée.

A cet effet, l'invention concerne également un ensemble comportant au moins une poignée de porte munie d'une béquille de manoeuvre de la poignée et d'un support de la béquille, l'ensemble comprenant en outre au moins un système de nettoyage et/ou de désinfection d'un tube creux tel que décrit ci-dessus, le tube creux constituant la béquille.

Des formes particulières de l'ensemble sont définies selon l'invention.

Avantageusement, l'ensemble comprend en outre un dispositif de verrouillage de la porte par action sur la béquille, ledit dispositif comportant un axe solidaire en rotation d'un carré de pêne demi-tour de la porte, et trois pièces montées autour de l'axe et formant chacune une liaison pivot avec l'axe, une première pièce formant une première liaison pivot avec l'axe ; une deuxième pièce étant fixée au support de la béquille et formant une deuxième liaison pivot avec l'axe, et une troisième pièce agencée entre les première et deuxième pièces et formant une troisième liaison pivot avec l'axe ; le dispositif comportant en outre un premier système de butées entre la première pièce et la deuxième pièce, de sorte à contraindre la première pièce dans son débattement angulaire ; et un second système de butées entre la troisième pièce d'une part et la première pièce ou la deuxième pièce d'autre part; le dispositif de verrouillage comportant en outre un système de transformation du mouvement pivot de la troisième pièce en un mouvement pivot sur un carré de pêne dormant de la porte, pour le verrouillage ou le déverrouillage de la porte.

Grâce au dispositif de verrouillage de la porte par action sur la béquille, un utilisateur peut verrouiller et déverrouiller la porte en actionnant uniquement la béquille de la poignée de porte. Ceci permet d'éviter à l'utilisateur de toucher un bouton de condamnation dédié, potentiellement souillé et infecté car non nettoyé par le système selon l'invention. Ceci évite ainsi à l'utilisateur tout risque de contamination potentielle lors du verrouillage ou du déverrouillage de la porte. Ainsi, grâce à un tel ensemble selon l'invention, une barrière intégrale aux transmissions bactériennes est avantageusement obtenue sur toute la séquence d'ouverture, de fermeture, de verrouillage et de déverrouillage de la porte.

Selon un deuxième aspect de l'invention, complémentaire mais indépendant du premier aspect, l'invention concerne également un dispositif de verrouillage de porte par action sur une béquille de manoeuvre d'une poignée de porte, la poignée comprenant en outre un support de béquille ; le dispositif comportant un axe solidaire en rotation d'un carré de pêne demi-tour de la porte, et trois pièces montées autour de l'axe et formant chacune une liaison pivot avec l'axe, une première pièce formant une première liaison pivot avec l'axe ; une deuxième pièce étant fixée au support de la béquille et formant une deuxième liaison pivot avec l'axe, et une troisième pièce agencée entre les première et deuxième pièces et formant une troisième liaison pivot avec l'axe ; le dispositif comportant en outre un premier système de butées entre la première pièce et la deuxième pièce, de sorte à contraindre la première pièce dans son débattement angulaire ; et un second système de butées entre la troisième pièce d'une part et la première pièce ou la deuxième pièce d'autre part ; le dispositif de verrouillage comportant en outre un système de transformation du mouvement pivot de la troisième pièce en un mouvement pivot sur un carré de pêne dormant de la porte, pour le verrouillage ou le déverrouillage de la porte. Cette solution avantageuse permet d'éviter à l'utilisateur de toucher un bouton de condamnation dédié, potentiellement souillé et infecté, et n'impose aucune modification ni ajout d'élément(s) additionnel(s) sur la porte elle-même, ni sur le bâti de la porte.

Avantageusement, la troisième pièce et le système de transformation de mouvement pivot sont configurés de sorte qu'un mouvement de la deuxième pièce dans un premier sens de rotation entraîne un mouvement de la troisième pièce et du système de transformation de mouvement pivot, pour le verrouillage de la porte, et qu'un mouvement de la deuxième pièce dans un second sens de rotation, opposé au premier sens, entraîne un mouvement de la première pièce et de l'axe, pour l'ouverture de la porte. Ceci permet à un utilisateur de pouvoir facilement et intuitivement ouvrir ou verrouiller la porte, par action uniquement sur la béquille de la poignée qui agit sur la deuxième pièce via le support de béquille. Par exemple, un mode de réalisation possible consiste en ce que l'utilisateur puisse ouvrir la porte via un mouvement de la béquille vers le bas qui agit sur le carré de pêne demi-tour de la porte ; et puisse verrouiller la porte via un mouvement de la béquille vers le haut qui agit sur le carré de pêne dormant de la porte. Une fois la porte verrouillée, le déverrouillage peut être effectué via un mouvement de la béquille vers le bas qui agit sur deuxième pièce, donc sur la première pièce via le premier système de butées, et donc sur la troisième pièce grâce au second système de butées.

Avantageusement, la première liaison pivot est configurée pour être sur le débattement angulaire de la poignée du côté de la porte opposé au côté d'installation du dispositif de verrouillage, de sorte qu'une inclinaison de la béquille du côté de la porte opposé au côté d'installation du dispositif, entraînant une rotation de l'axe, est découplée de tout mouvement sur la première pièce. Ceci permet d'empêcher tout déverrouillage intempestif de la porte par action sur la béquille du côté opposé au côté d'installation du dispositif de déverrouillage. Lorsque l'utilisateur a verrouillé la porte de son côté, une tierce personne ne peut ainsi pas déverrouiller la porte en inclinant la poignée, notamment en l'inclinant vers le bas.

### Brève description des figures

Les buts, avantages et caractéristiques du système de nettoyage et/ou de désinfection d'un tube creux selon l'invention, ainsi que de l'ensemble le comprenant, apparaîtront mieux dans la description suivante sur la base d'au moins une forme d'exécution non limitative illustrée par les dessins sur lesquels :
[Fig. 1] est une vue en perspective d'un ensemble comportant une poignée de porte ainsi qu'un système de nettoyage et/ou de désinfection d'un tube creux constituant la béquille de la poignée, et un dispositif de verrouillage de porte par action sur la béquille, selon l'invention ;
[Fig. 2] est une vue en perspective du système de nettoyage et/ou de désinfection de la figure 1, le système comprenant une béquille, une bague et un réservoir de liquide ;
[Fig. 3] est une vue analogue à celle de la figure 2, montrant l'intérieur de la béquille, et notamment un dispositif d'actionnement électromécanique de la bague ;
[Fig. 4] est une vue de l'arrière du système de la figure 2, vu selon une direction D1 d'extension longitudinale de la béquille ;
[Fig. 5] est une vue de dessous du système de la figure 2 ;
[Fig. 6] est une vue en perspective du dispositif d'actionnement électromécanique de la figure 3 ;
[Fig. 7] est une vue en perspective de la bague et du réservoir de la figure 2 ;
[Fig. 8] est une vue en perspective de la béquille de la figure 2 ;
[Fig. 9] est une vue en perspective du dispositif de verrouillage de porte de la figure 1, le dispositif comportant un support de fixation, un système mécanique et un ensemble à engrenages ;
[Fig. 10] est une vue analogue à celle de la figure 9, dans laquelle le support de fixation a été masqué ;
[Fig. 11] est une vue en perspective du système mécanique de la figure 9, le système mécanique comprenant un axe et trois pièces montées autour de l'axe ;
[Fig. 12] est une vue en perspective du système mécanique de la figure 10, dans laquelle une des trois pièces a été masquée ;
[Fig. 13] est une vue en perspective de l'axe de la figure 10 ;
[Fig. 14] est une vue en perspective d'une première pièce de la figure 10 ;
[Fig. 15] est une vue en perspective d'une deuxième pièce de la figure 10 ; et
[Fig. 16] est une vue en perspective d'une troisième pièce de la figure 10.

### Description détaillée de l'invention

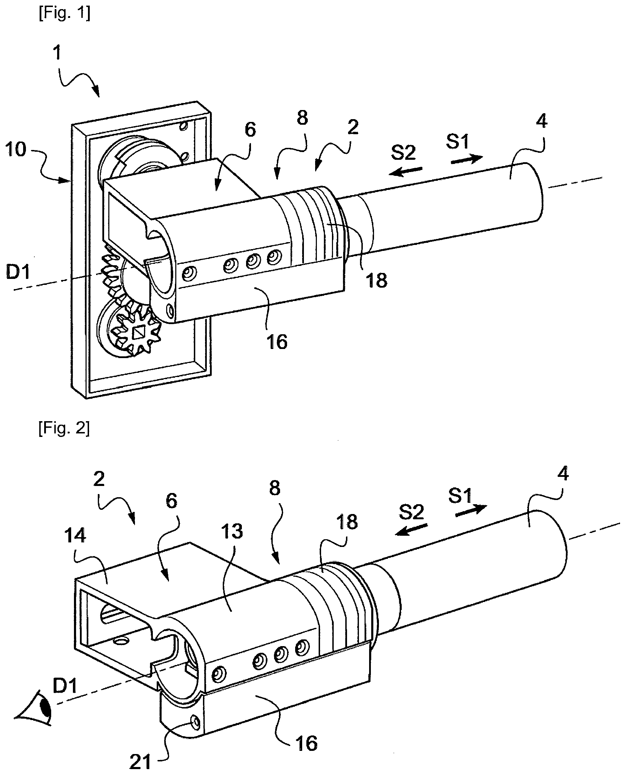

La figure 1 représente un ensemble 1 comportant au moins une poignée de porte 2 munie d'une béquille 4 de manoeuvre de la poignée et d'un support 6 de la béquille 4. L'ensemble 1 comporte en outre au moins un système 8 de nettoyage et/ou de désinfection d'un tube creux 4 constituant la béquille, et un dispositif 10 de verrouillage de la porte par action sur la béquille 4. En variante, et bien qu'une seule poignée 2 et un seul système 8 soient représentés sur la figure 1 et sur les figures suivantes, l'ensemble 1 peut aussi comporter deux poignées 2 et deux systèmes de nettoyage et/ou de désinfection 8, chaque système 8 étant dédié au nettoyage et/ou à la désinfection d'une des béquilles 4 liée à une poignée respective 2. Dans ce cas de figure, chaque poignée 2 est agencée d'un côté respectif de la porte. En variante encore, le système 8 est adapté au nettoyage et/ou à la désinfection de tout tube creux 4, pouvant par exemple être un tube d'une barre équipant un moyen de transport de type bus ou métro, une poignée de type bâton de maréchal ou encore une rampe telle que par exemple une rampe d'escalier. Sans que cela ne soit limitatif dans le cadre de la présente invention, le tube creux 4 présente par exemple un diamètre compris entre 30 mm et 50 mm.

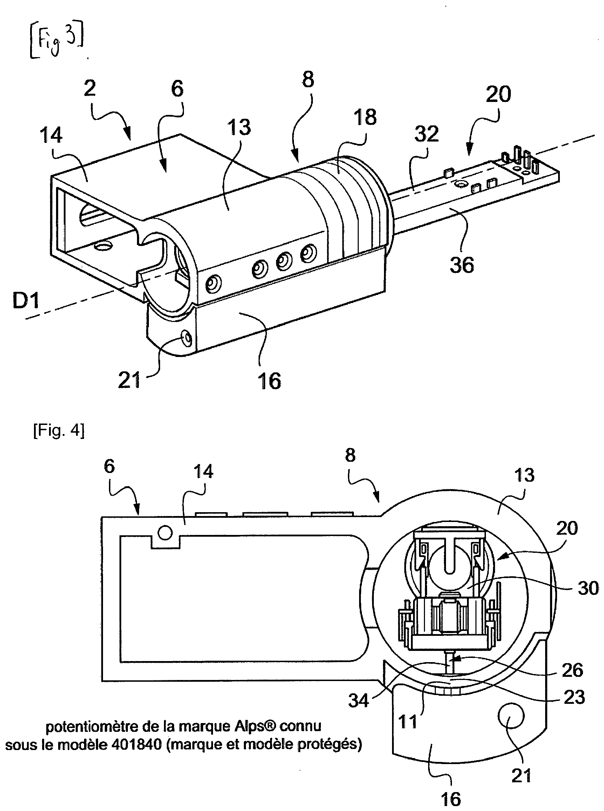

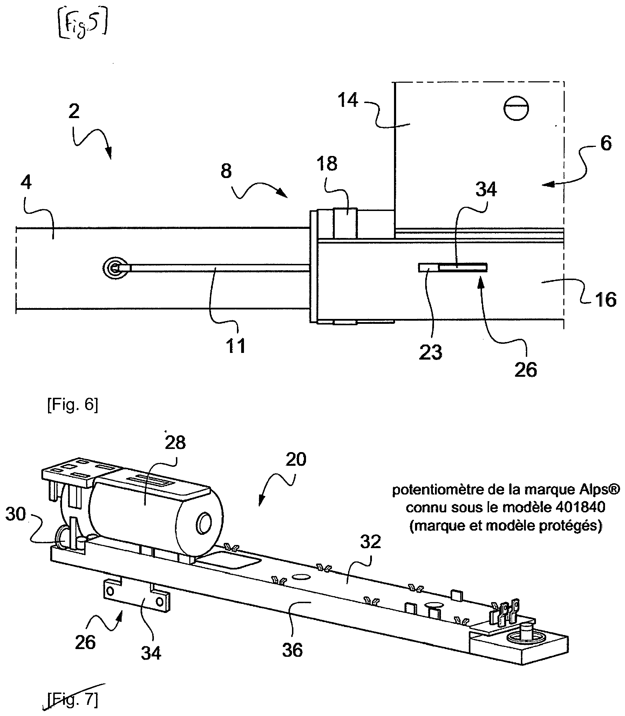

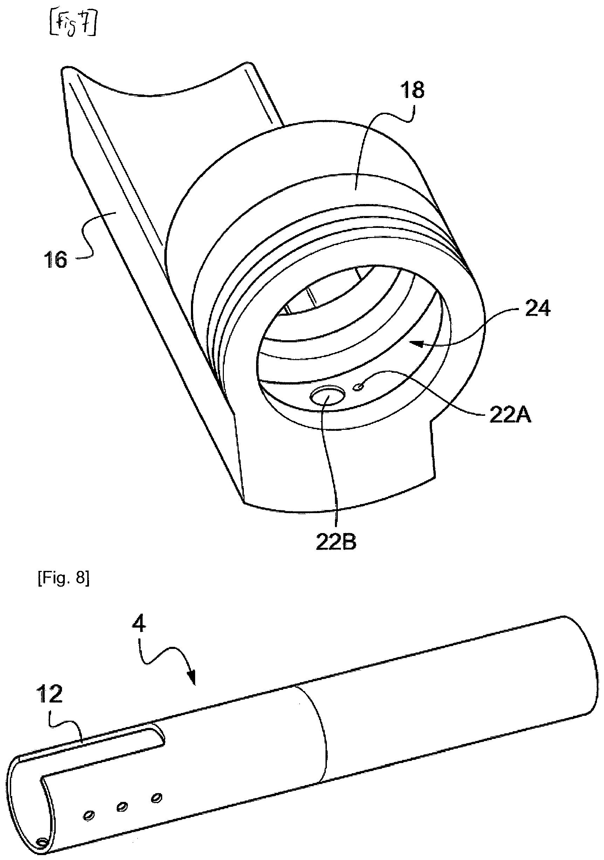

La béquille 4 est par exemple de forme cylindrique. La béquille 4 s'étend dans sa plus grande dimension selon une direction longitudinale D1, et est munie d'une fente longitudinale 11 pour permettre le passage d'un élément d'entrainement en translation, comme cela sera détaillé par la suite. Une telle fente 11, qui s'étend par exemple sur le dessous de la béquille 4 pour des raisons d'esthétique, est visible sur les figures 4 et 5. La longueur de la fente longitudinale 11, mesurée selon la direction longitudinale D1, est par exemple comprise entre 80 mm et 130 mm. Par ailleurs, selon un exemple de réalisation particulier représenté sur la figure 8, la béquille 4 peut en outre comporter une découpe longitudinale 12 permettant l'introduction d'un dispositif d'actionnement électromécanique 20 au sein de la béquille 4. De préférence, la béquille 4 ou la bague 18 est munie d'au moins un marquage visuel, un tel marquage visuel n'étant pas visible sur les figures pour des raisons de clarté. Un tel marquage visuel prend par exemple la forme d'un marquage coloré sur la surface extérieure de la béquille 4 ou sur la face supérieure de la bague 18, ou encore d'un repère gravé sur cette surface extérieure ou sur cette face supérieure, et est indicatif d'une rupture en liquide et/ou en alimentation électrique. Sans que cela ne soit limitatif dans le cadre de la présente invention, la béquille 4 présente par exemple un diamètre compris entre 20 mm et 27 mm et une longueur totale, mesurée selon la direction longitudinale D1, comprise entre 150 mm et 200 mm. La béquille 4 est par exemple constituée d'un alliage métallique, tel que par exemple de l'acier inoxydable.

Le support 6 de béquille est classiquement monté mobile en rotation par rapport à la porte, et notamment par rapport à un dispositif support fixé sur celle-ci, tel que par exemple le dispositif de verrouillage 10 dans l'exemple illustratif de la figure 1. En variante non représentée, le dispositif support peut par exemple être une rosace de porte classique. Le débattement angulaire du support 6 est typiquement compris entre 0 et 60 degrés, le débattement étant mesuré par rapport à une direction sensiblement horizontale lorsque l'ensemble 1 est fixé à une porte. Le support 6 est solidaire en rotation d'une pièce elle-même reliée à un carré de pêne demi-tour de la porte, comme cela sera détaillé par la suite, pour permettre l'ouverture ou la fermeture de la porte. Dans un exemple de réalisation particulier visible sur les figures 1 à 3, le support 6 est constitué en une seule pièce de matière monobloc. Cette pièce monobloc comprend par exemple une première partie 13 sensiblement tubulaire, et une deuxième partie 14 sensiblement parallélépipédique. La béquille 4 est en partie reçue dans la première partie tubulaire 13, et est fixée à celle-ci, par exemple au moyen d'une ou plusieurs vis. La pièce monobloc constituant le support 6 est par exemple en matière plastique ou métallique.

Comme illustré sur les figures 2 à 5, le système 8 de nettoyage et/ou de désinfection de la béquille 4 comprend, outre la béquille 4, une bague 18 renfermant un tampon absorbant imprégné d'un liquide désinfectant et/ou dégraissant, et un dispositif 20 d'actionnement électromécanique de la bague 18. Le tampon absorbant n'est pas représenté sur les figures pour des raisons de clarté.

Selon un mode de réalisation particulier, représenté sur les figures 1 à 5 et 7, le système 8 comprend également un réservoir 16 de liquide désinfectant et/ou dégraissant.

Selon un autre mode de réalisation, non représenté sur les figures, le système 8 ne comporte pas de réservoir 16 de liquide désinfectant et/ou dégraissant. Dans ce mode de réalisation, le tampon absorbant est agencé autour de la béquille 4, et entoure au moins partiellement cette dernière. Le tampon absorbant, qui présente par exemple une forme sensiblement circulaire ou annulaire, est ainsi agencé entre la béquille 4 et la bague 18, et est solidaire de la bague 18. Dans ce cas, la bague 18 comporte par exemple une première partie sensiblement annulaire, dans laquelle est agencé le tampon, et une deuxième partie sous forme de languette longitudinale s'étendant selon la direction D1, et munie d'une fente longitudinale permettant l'insertion et la solidarisation d'un élément d'entrainement en translation 26. De préférence, le tampon absorbant entoure complètement la béquille 4, permettant d'obtenir un nettoyage de la béquille 4 sur toute sa circonférence. Le tampon absorbant présente des capacités d'absorption élevées, par exemple en étant capable d'absorber une quantité de liquide sensiblement égale à quatre fois le poids du tampon à vide. Pour la recharge du système 8 en liquide désinfectant et/ou dégraissant, un utilisateur du système 8 peut ainsi imbiber directement le tampon absorbant au moyen d'un flacon applicateur ou d'une seringue par exemple. Ceci permet avantageusement de se dispenser de toute recharge d'un réservoir connexe, et de faciliter ainsi l'opération de recharge en liquide. Le tampon absorbant est typiquement en matière textile, telle que par exemple de la feutrine synthétique polyester ou naturelle en laine. Lorsque le tampon absorbant présente une forme annulaire, le tampon absorbant présente par exemple un diamètre intérieur compris entre 25 mm et 30 mm, un diamètre extérieur compris entre 35 mm et 40 mm, et une longueur, mesurée selon la direction longitudinale D1, comprise entre 10 mm et 30 mm.

Dans le mode de réalisation particulier représenté sur les figures 1 à 5 et 7, le réservoir 16 est configuré pour contenir un liquide désinfectant et/ou dégraissant. Un tel liquide est par exemple un liquide à base alcoolique, ou encore un liquide à base d'eau oxygénée intégrant des ions d'argent, sans que cela ne soit limitatif dans le cadre de la présente invention. Une ouverture de remplissage 21 est par exemple prévue sur une surface extérieure du réservoir 16, afin de permettre le remplissage du réservoir 16 par un utilisateur, par exemple au moyen d'un flacon applicateur (non représenté).

La bague 18 est montée coulissante sur la béquille 4, le long de la direction longitudinale D1. La bague 18 est apte à coulisser selon les deux sens de translation S1, S2 visibles sur les figures 1 et 2, le long de cette direction D1. La bague 18 comprend par exemple une première partie de forme sensiblement annulaire, et une deuxième partie longitudinale. Dans ce cas, la béquille 4 est agencée au centre de l'anneau formant la première partie de la bague 18, comme illustré sur les figures 1 et 2. Dans le mode de réalisation particulier représenté sur les figures 1 à 5 et 7, la bague 18 est solidarisée au réservoir 16. Plus précisément, la deuxième partie longitudinale de la bague 18 renferme le réservoir 16. Selon un exemple de réalisation particulier illustré notamment sur les figures 4 et 5, une fente longitudinale 23 est ménagée dans la bague 18 pour permettre l'insertion et la solidarisation d'un élément d'entrainement en translation 26, comme cela sera détaillé par la suite. La fente 23 s'étend par exemple sur le dessous de la deuxième partie longitudinale de la bague 18, typiquement sur une partie de la longueur de celle-ci. La longueur de la fente longitudinale 23, mesurée selon la direction longitudinale D1, est par exemple comprise entre 20 mm et 25 mm.

Sans que cela ne soit limitatif dans le cadre de la présente invention, la première partie annulaire de la bague 18 présente par exemple un diamètre compris entre 35 mm et 50 mm et une longueur, mesurée selon la direction longitudinale D1, comprise entre 20 mm et 35 mm.

De préférence, comme représenté dans l'exemple illustratif des figures 1 à 5 et 7, le réservoir 16 et la bague 18 forment une seule et même pièce monobloc. Une telle pièce monobloc est par exemple obtenue via un procédé de fabrication par impression en trois dimensions, aussi appelé impression 3D. La pièce monobloc constituant le réservoir 16 et la bague 18 est par exemple en matière plastique. De préférence, le réservoir 16 présente une contenance par exemple sensiblement égale à 10 mL. Toutefois, d'autres contenances sont envisageables pour le réservoir 16, allant par exemple de 10 mL à 30 mL. En particulier, grâce au procédé d'impression 3D utilisé pour la fabrication du réservoir 16 et de la bague 18, l'utilisateur final peut avantageusement opter pour la contenance de son choix pour le réservoir 16, en fonction notamment des contraintes d'autonomie désirées.

Le tampon absorbant est agencé autour de la béquille 4, et entoure au moins partiellement cette dernière. Le tampon absorbant, qui présente par exemple une forme sensiblement circulaire ou annulaire, est ainsi agencé entre la béquille 4 et la bague 18, et est solidaire de la bague 18. De préférence, le tampon absorbant entoure complètement la béquille 4, permettant d'obtenir un nettoyage de la béquille 4 sur toute sa circonférence. Lorsque le système 8 comprend un réservoir 16, le tampon absorbant est en communication fluidique avec ce dernier. Plus précisément, selon un exemple de réalisation préférentiel illustré sur la figure 7, un orifice 22A est ménagé dans la bague 18 en regard du tampon absorbant. L'orifice 22A débouche dans le réservoir 16. Le réservoir 16 et l'orifice 22A sont configurés pour que du liquide s'écoule depuis le réservoir 16 vers le tampon, à travers l'orifice 22A, lorsque la béquille 4 est inclinée vers le bas. De par l'inclinaison de la béquille 4 lors de sa manipulation par un utilisateur, du liquide contenu dans le réservoir 16 peut sortir par gravité de l'orifice 22A et venir humecter le tampon absorbant. Ceci permet d'humecter rapidement le tampon, par exemple au bout de quelques secondes après l'inclinaison de la béquille 4, suite à une manipulation de la poignée 2. Cette méthode de recharge du tampon par arrosage correspond à un cas d'utilisation où la poignée 2 est sollicitée de manière soutenue, par exemple lors de périodes pendant les heures de pointe, dans une utilisation quotidienne et/ou diurne de la poignée 2.

En variante ou en complément, et comme illustré sur la figure 7, un autre orifice 22B est ménagé dans la bague 18 en regard du tampon absorbant. L'orifice 22B débouche dans le réservoir 16, et le système 8 comporte en outre une mèche capillaire, une telle mèche n'étant pas représentée sur les figures pour des raisons de clarté. La mèche capillaire est agencée à travers l'orifice 22B jusque dans le réservoir 16, et est en contact avec le tampon absorbant, pour permettre un transfert de liquide par capillarité du réservoir 16 vers le tampon absorbant. Ceci permet de garantir un tampon imbibé de liquide sans qu'il n'y ait besoin de manipuler la poignée 2. Cette méthode de recharge du tampon par capillarité correspond à un cas d'utilisation où la poignée est peu sollicitée et/ou le tampon n'est plus alimenté par arrosage, par exemple lors de périodes creuses d'utilisation telles que des périodes nocturnes. Même si aucune manipulation de la poignée 2 n'a eu lieu durant une dizaine d'heures pendant la nuit, cette méthode de recharge par capillarité permet de garantir d'avoir un tampon humide au petit matin.

De préférence, et comme illustré sur la figure 7, le tampon est reçu dans la bague 18 au niveau d'un emplacement 24, lequel emplacement 24 est décalé axialement de la liaison mécanique entre la bague 18 et un élément 26 d'entrainement en translation prévu sur le dispositif d'actionnement électromécanique 20. Ceci permet de faire coïncider la course du dispositif d'actionnement 20 avec la course de la bague 18, et d'ainsi nettoyer la bonne longueur de béquille tout en utilisant tout le débattement possible du dispositif 20. Le tampon absorbant peut être constitué de tout type de matériau absorbant et/ou capillaire. Par exemple, et sans que cela ne soit limitatif dans le cadre de la présente invention, le tampon absorbant peut être en matière textile, telle que par exemple de la feutrine synthétique polyester ou naturelle en laine.

Comme illustré sur les figures 3, 4 et 6, le dispositif d'actionnement électromécanique 20 est agencé à l'intérieur du tube creux constituant la béquille 4, et comprend un élément 26 d'entrainement en translation longitudinale. L'élément 26 est visible sur les figures 4 et 6. De préférence, comme illustré sur les figures 4 et 6, le dispositif 20 comprend en outre un moteur 28 alimenté électriquement, et un ensemble poulies/courroies 30 actionné par le moteur 28. De préférence encore, le dispositif 20 comprend en outre un microcontrôleur de commande du moteur 28, relié au moteur 28 ; ainsi qu'un inclinomètre relié au microcontrôleur. De préférence encore, le dispositif 20 comprend en outre un hacheur relié au moteur 28, au microcontrôleur, et à la ou aux pile(s) d'alimentation. Le microcontrôleur, l'inclinomètre et le hacheur, qui ne sont pas représentés sur les figures pour des raisons de clarté, se présentent typiquement sous la forme de composants électroniques connectés sur une carte de circuit imprimé 32.

Comme illustré sur la figure 4, l'élément 26 d'entrainement en translation longitudinale fait saillie à travers la fente longitudinale 11 de la béquille 4 et est lié mécaniquement à la bague 18. Pour ce faire, et selon l'exemple de réalisation particulier illustré sur les figures 1 à 6, l'élément 26 d'entrainement en translation longitudinale est par exemple inséré au travers de la fente longitudinale 23, afin de permettre la liaison mécanique avec la bague 18. L'élément 26 est ainsi configuré pour permettre le déplacement de la bague 18 en translation longitudinale le long de la béquille 4, plus précisément le long de la direction longitudinale D1. Selon cet exemple de réalisation particulier, l'élément 26, qui est typiquement un charriot mobile muni d'une languette 34, est en outre relié à l'ensemble poulies/courroies 30, comme illustré sur la figure 6. La languette 34 fait saillie à travers les fentes longitudinales respectives 11, 23 de la béquille 4 et de la bague 18, et permet l'entrainement en translation longitudinale de la bague 18 le long de la béquille 4, la fente longitudinale 23 de la bague 18 étant plus courte que la fente longitudinale 11 de la béquille 4. Comme illustré sur la figure 6, la languette 34 présente par exemple une forme en « T ».

Le dispositif d'actionnement électromécanique 20 est un potentiomètre linéaire motorisé. Dans le cas où l'élément d'entrainement en translation longitudinale 26 est un charriot mobile, le charriot mobile 26 est monté en translation sur une partie fixe 36 du potentiomètre. Un tel potentiomètre linéaire comprend une piste électriquement résistante et un curseur mobile le long de la piste. Dans le cas où l'élément d'entrainement en translation longitudinale 26 est un charriot mobile, la languette 34 du charriot mobile 26 forme le curseur. La piste résistante se termine à chacune de ses extrémités par deux bornes, une des deux bornes étant reliée à un potentiel électrique haut, l'autre borne étant reliée à un potentiel électrique bas. Une troisième borne est reliée au curseur 34. Ainsi, la valeur de la résistance mesurée entre la troisième borne et une des deux autres bornes varie proportionnellement à la distance entre les bornes d'extrémité de la piste et le curseur 34. Ceci permet d'obtenir un retour potentiométrique avantageux dans le cadre de la présente invention, car permettant d'obtenir une information précise quant à la position du curseur 34 et donc de la bague 18. Ceci permet notamment de garantir que le mouvement de la bague 18, et donc le nettoyage de la béquille 4, s'est effectivement effectué sur toute la longueur de la béquille 4. Le potentiomètre linéaire motorisé 20 présente une course par exemple sensiblement égale à 100 mm. Sans que cela ne soit limitatif dans le cadre de la présente invention, le potentiomètre linéaire motorisé 20 représenté sur les figures 4 et 6 est un potentiomètre de la marque Alps^{®} connu sous le modèle 401840 (marque et modèle protégés).

Le moteur 28 est par exemple alimenté électriquement via une ou plusieurs piles (non représentées sur les figures pour des raisons de clarté). La ou les pile(s) peuvent être des piles rechargeables ou non rechargeables. L'utilisation de piles rechargeables, qui peuvent par exemple être rechargées via un cordon de type USB (de l'anglais Universal Serial Bus), permet d'offrir un bilan économique et écologique vertueux. Typiquement, de telles piles rechargeables peuvent être choisies parmi des piles polymères lithium-ion Li-Po (de l'anglais Lithium Polymer), ou encore des piles nickel-hydrure métallique NiMH (de l'anglais Nickel-Metal Hydride). Les piles Li-Po présentent une grande capacité mais nécessitent de prévoir dans le système 8 un compartiment à piles ignifugé et résistant à l'implosion. Les piles NiMH présentent une sécurité d'utilisation accrue mais nécessitent le recours à un chargeur externe. De préférence, la tension d'alimentation fournie par la ou les pile(s), qu'elle(s) soient rechargeable(s) ou non, est sensiblement égale à 9 V. Dans le cas de piles non rechargeables, ces dernières sont par exemple choisies de manière à stocker une énergie comprise entre sensiblement 0,4 Ah et 0,6 Ah. Ceci conduit à une durée de vie estimée sensiblement égale à 4000 cycles de nettoyage, ce qui, ramené à un établissement recevant du public, établit la durée de vie à environ 3 mois, en condition forcée d'utilisation. Dans le cas de piles rechargeables, la durée de vie estimée avant recharge est sensiblement égale à 3500 cycles de nettoyage. Le moteur 28 est typiquement un moteur à courant continu à balais, sans que cela ne soit limitatif dans le cadre de la présente invention.

L'ensemble poulies/courroies 30 est entrainé par le moteur 28, et est configuré pour déplacer en translation, le long de la direction longitudinale D1, l'élément 26 d'entrainement en translation longitudinale.

Selon un exemple de réalisation préférentiel, le microcontrôleur comprend des moyens de traitement et des moyens mémoire stockant un code source. Le code source comporte des instructions de programme qui, lorsqu'exécutées par les moyens de traitement, déclenchent une séquence de commande du moteur, pour le déplacement de la bague 18 en translation longitudinale le long de la béquille 4. De préférence, le microcontrôleur comprend en outre un compteur pré-étalonné.

De préférence, une phase d'accélération au départ et de décélération à l'arrivée de la bague 18 est implémentée dans les instructions de programme du code source. Ceci permet d'éviter au système 8 selon l'invention tout bruit inhérent aux « coups de bélier » liés aux fins de course de la bague 18. De préférence encore, le microcontrôleur est configuré de manière à permettre de sélectionner et/ou paramétrer une stratégie de nettoyage souhaitée. Plus précisément, la stratégie de nettoyage, sélectionnée et/ou paramétrée via le code source, peut permettre de modifier des paramètres tels que par exemple la vitesse de mouvement, la fréquence de mouvement, et/ou les valeurs d'accélération et de décélération de la bague 18 en début et en fin de mouvement. Le code source du système 8 peut ainsi, par exemple, être paramétré de manière à ce que le système 8 nettoie et/ou désinfecte la béquille 4 à une fréquence donnée même si la béquille 4 n'a pas été sollicitée.

De préférence encore, le code source est paramétré de manière à ce que le système 8, en particulier le dispositif d'actionnement électromécanique 20, repositionne la bague 18 à sa position de départ lorsque celle-ci rentre en contact avec la main d'une personne. Un tel système de retour anti-pincement est par exemple rendu possible par une comparaison, effectuée par le microcontrôleur, de la position mesurée de la bague 18, une telle mesure de position étant obtenue via le retour potentiométrique offert par le potentiomètre 20. Avantageusement, le code source peut par exemple être paramétré de sorte que la bague 18 revienne à sa position de départ à une vitesse accélérée, correspondant à une durée de 1 seconde au lieu de 2 secondes en régime normal, afin de limiter la gêne occasionnée par la présence de la bague 18 auprès de l'utilisateur final.

Le compteur pré-étalonné enregistre un nombre prédéterminé de cycles du système 8, correspondant à une rupture en liquide et/ou en alimentation électrique. Le nombre de cycles prédéterminés est par exemple déterminé par des études préalables d'étalonnage. De préférence, la séquence de commande du moteur 28 est paramétrée au sein du code source de sorte que, lorsque le compteur a atteint le nombre prédéterminé de cycles, le microcontrôleur commande le moteur 28 pour amener la bague 18, en fin de mouvement, jusqu'à une position prédéfinie sur la béquille 4 laissant apparaître le marquage visuel, soit sur la béquille 4 directement, soit sur le dessus de la bague 18. Ceci permet d'avertir de manière fiable le personnel de maintenance quant à la nécessité d'une recharge en liquide et/ou en alimentation électrique, ou à la nécessité de remplacer les piles d'alimentation lorsque celles-ci sont non rechargeables. En outre, une telle solution permet de s'affranchir de l'utilisation de capteurs et/ou de détecteurs pour déterminer la rupture en liquide et/ou en énergie électrique.

Le microcontrôleur de commande du moteur 28 est ainsi avantageusement configuré pour permettre une gestion efficace de l'autonomie en liquide et/ou en alimentation électrique du système, ainsi qu'une sélection par l'utilisateur des conditions et/ou de la stratégie de nettoyage désirée(s). Le microcontrôleur se présente par exemple sous la forme d'une puce électronique connectée sur la carte de circuit imprimé 32. Le microcontrôleur est par exemple choisi de sorte à présenter une très faible consommation d'énergie en mode veille, de l'ordre de quelques nanoampères, typiquement une consommation d'énergie sensiblement égale à 50 nA. Ceci permet d'augmenter la durée de vie des piles d'alimentation.

L'inclinomètre est configuré pour détecter un mouvement de la béquille 4, typiquement un mouvement de retour de la béquille 4 à sa position horizontale initiale. En variante, le mouvement de la béquille 4 détecté par l'inclinomètre peut être un mouvement vers le bas, correspondant à une ouverture de la porte. L'inclinomètre est en outre configuré pour transmettre au microcontrôleur un signal de détection correspondant. L'inclinomètre est par exemple constitué d'un tube comprenant une bille apte à se déplacer dans le tube, ainsi qu'un interrupteur de contact. La bille est réalisée en un matériau conducteur électrique et est apte à se déplacer à l'intérieur du tube lorsque la poignée 2 est actionnée. L'interrupteur est relié au microcontrôleur de sorte à pouvoir lui transmettre le signal de détection lorsque l'interrupteur est fermé par le contact de la bille. Cette interruption au niveau du microcontrôleur vient réveiller ce dernier. A l'issue d'une durée prédéfinie écoulée depuis la réception du signal de détection, le microcontrôleur est configuré pour transmettre au moteur 28, via le hacheur, un signal de commande d'un mouvement de va-et-vient de la bague 18 le long de la béquille 4. Le microcontrôleur retourne ensuite dans son mode veille. La durée prédéfinie est par exemple comprise entre une seconde et trois secondes. Le microcontrôleur est par exemple configuré de sorte à ce que la commande d'un mouvement de va-et-vient de la bague 18 corresponde à un va-et-vient effectif d'une durée comprise typiquement entre une seconde et quatre secondes. Une telle durée permet de bien appliquer le liquide sur toute la surface et toute la longueur de la béquille 4.

Le hacheur est adapté pour piloter le moteur 28. Le hacheur est par exemple choisi de sorte à présenter une très faible consommation d'énergie en mode veille, typiquement une consommation d'énergie sensiblement égale à 80 nA. Ceci permet d'augmenter la durée de vie des piles d'alimentation.

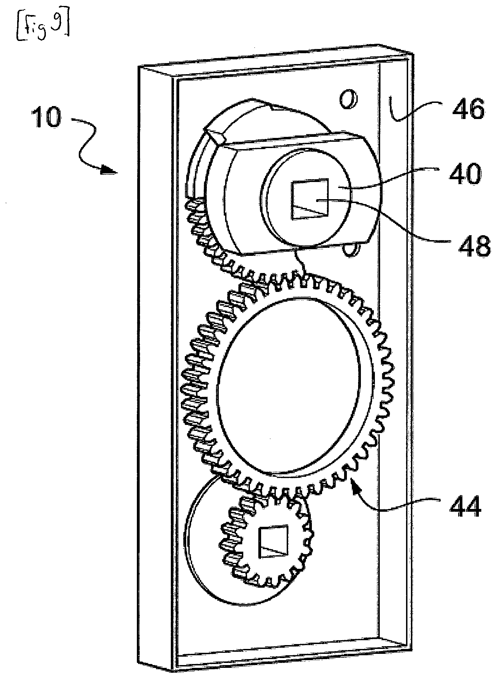

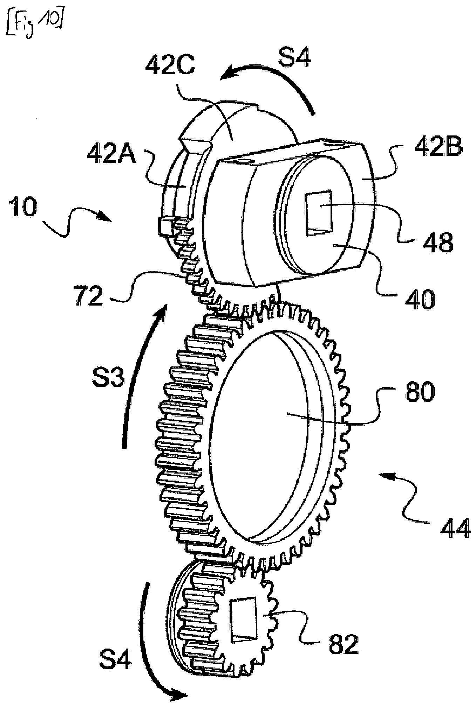

Les figures 9 et 10 représentent un dispositif 10 de verrouillage de porte par action sur une béquille 4 de manoeuvre d'une poignée de porte 2, par exemple intégré avantageusement au sein de l'ensemble 1 de la figure 1. En variante, le dispositif de verrouillage 10 peut équiper toute poignée de porte 2 munie d'une béquille de manoeuvre 4 et d'un support de béquille 6, et non forcément munie du système de nettoyage et/ou de désinfection 8.

Le dispositif de verrouillage 10 comporte un axe 40 ; trois pièces 42A, 42B, 42C montées autour de l'axe 40 et formant chacune une liaison pivot avec l'axe 40 ; et un système 44 de transformation du mouvement pivot d'une des pièces 42C en un mouvement pivot sur un carré de pêne dormant de la porte. Un tel carré de pêne dormant n'est pas représenté sur les figures pour des raisons de clarté. Le système de transformation du mouvement pivot 44 permet, sous l'effet d'un mouvement de rotation de la pièce 42C, le verrouillage ou le déverrouillage de la porte. De préférence, comme illustré sur la figure 9, le dispositif de verrouillage 10 comporte un outre un support de fixation 46 destiné à être fixé à la porte.

L'axe 40 est solidaire en rotation d'un carré de pêne demi-tour de la porte, un tel carré de pêne demi-tour n'étant pas représenté sur les figures pour des raisons de clarté. Comme visible sur les figures 9 à 13, l'axe 40 est percé, selon sa direction principale d'extension D2, d'une ouverture 48 de réception du carré de pêne demi-tour. Pour la suite de la description, la direction principale d'extension D2 de l'axe 40 sera appelée direction longitudinale, et toute direction perpendiculaire à la direction longitudinale D2 sera appelée direction transversale. L'ouverture 48 présente une forme complémentaire de celle du carré de pêne demi-tour afin d'assurer la solidarisation en rotation, par exemple une forme sensiblement rectangulaire ou carrée dans l'exemple illustratif de ces figures.

Dans l'exemple de réalisation particulier représenté sur les figures 9 à 13, l'axe 40 est muni, sur sa surface extérieure, d'au moins une fente transversale 50. De préférence, l'axe 40 comprend deux fentes transversales 50, disposées à sensiblement 180 degrés l'une de l'autre sur la surface extérieure de l'axe 40.

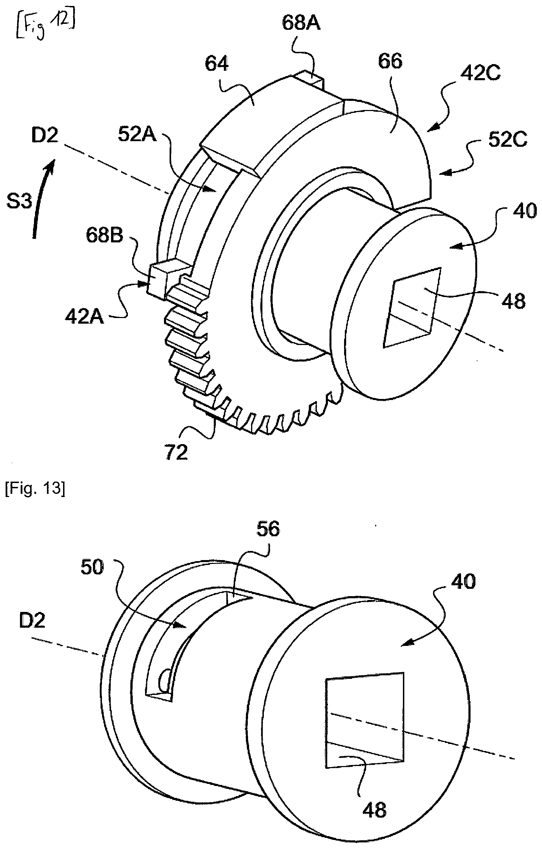

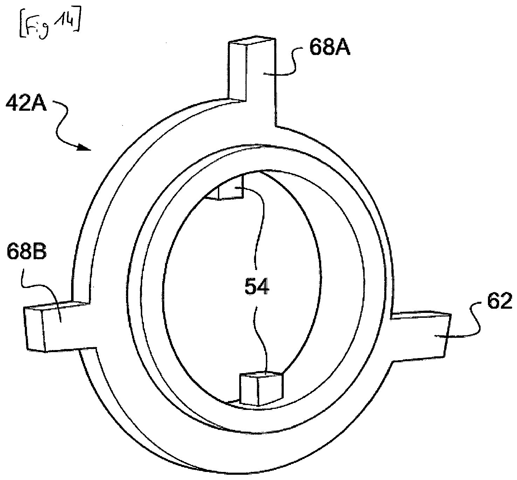

Une première pièce 42A forme une première liaison pivot 52A avec l'axe 40. Pour ce faire, dans un exemple de réalisation particulier représenté sur les figures 13 et 14, la première pièce 42A présente une forme sensiblement annulaire et présente au moins une butée transversale 54 faisant saillie sur un pourtour intérieur de l'anneau. De préférence, comme visible sur la figure 14, la première pièce 42A comprend deux butées transversales 54, disposées à sensiblement 180 degrés l'une de l'autre sur le pourtour intérieur de l'anneau formé par la première pièce 42A. La partie centrale vide de l'anneau permet le passage de l'axe 40. Chaque butée transversale 54 est ainsi insérée dans une des fentes transversales 50 de l'axe 40. Sous l'effet de la rotation de la première pièce 42A autour de l'axe 40, chaque butée transversale 54 est apte à se déplacer dans une fente transversale correspondante 50 jusqu'à une position d'appui sur un bord intérieur 56 de l'axe 40, permettant d'entraîner l'axe 40 et donc le pêne demi-tour en rotation. En variante non représentée, la ou les fente(s) 50 ménagées sur l'axe 40 peuvent être remplacées par des butées, et la ou les butée(s) transversale(s) 54 ménagées sur la première pièce 42A peuvent être remplacées par des lumières, complémentaires des butées ménagées sur l'axe 40.

Dans un mode de réalisation préférentiel représenté sur les figures 9 à 14, la première liaison pivot 52A est configurée pour être sur le débattement angulaire de la poignée 2 du côté de la porte opposé au côté d'installation du dispositif de verrouillage 10. Ainsi, une inclinaison de la béquille 4 du côté de la porte opposé au côté d'installation du dispositif 10, entraînant une rotation de l'axe 40, est découplée de tout mouvement sur la première pièce 42A. Pour ce faire, dans l'exemple de réalisation particulier représenté sur les figures 13 et 14, chaque fente transversale 50 est configurée de telle sorte qu'une inclinaison de la béquille 4 du côté de la porte opposé au côté d'installation du dispositif de verrouillage 10, entraînant une rotation de l'axe 40, est découplée de tout mouvement sur la butée correspondante 54 de la première pièce 42A. Ce découplage a pour conséquence qu'en position de verrouillage du dispositif (obtenue par exemple suite à l'actionnement de la béquille 4 par un utilisateur à l'intérieur d'une pièce de type cabinet de toilettes ou cabinet d'aisance), une tierce personne qui incline la poignée 2 côté extérieur fait pivoter le carré de pêne demi-tour de la porte, et donc l'axe 40, mais sans entraîner en rotation les butées transversales 54 de la première pièce 42A, et donc sans agir sur la troisième pièce 42C liée au verrouillage et au déverrouillage de la porte. Ceci permet d'empêcher tout déverrouillage intempestif de la porte par action sur la béquille 4 du côté opposé au côté d'installation du dispositif 10. Lorsqu'un utilisateur a verrouillé la porte de son côté par action sur la béquille 4, une tierce personne ne peut ainsi pas déverrouiller la porte en inclinant la poignée 2, notamment en l'inclinant vers le bas. Afin de respecter les normes de sécurité actuelles, il lui est toutefois possible de déverrouiller d'urgence le système de la même manière que les standards actuels, à savoir au moyen d'une pièce de monnaie ou d'un tournevis.

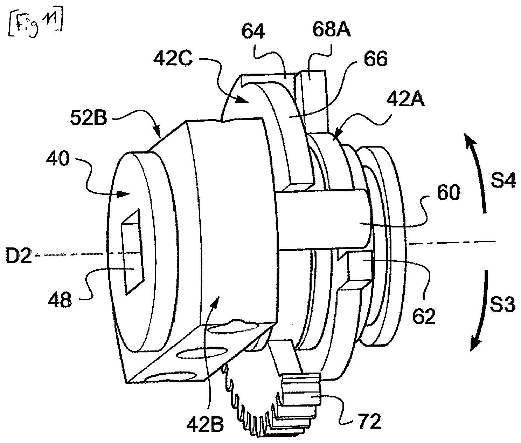

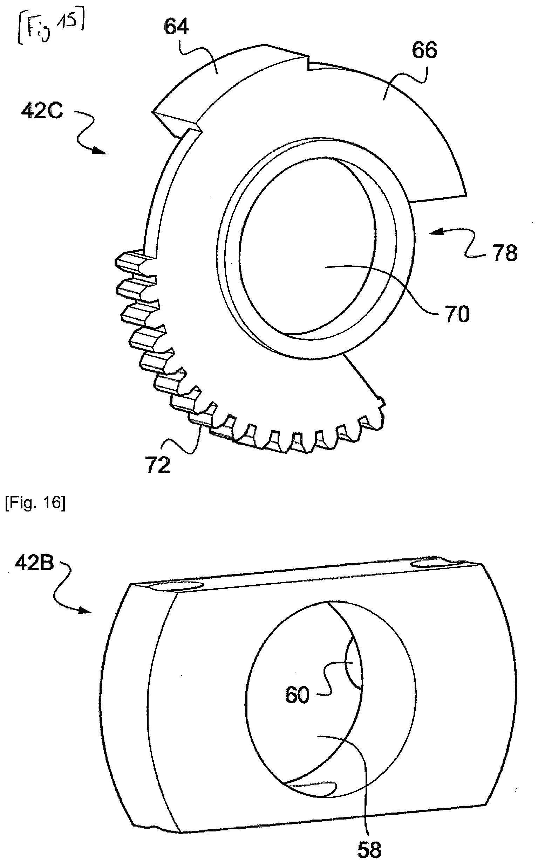

Une deuxième pièce 42B forme une deuxième liaison pivot 52B avec l'axe 40. Lorsque le dispositif de verrouillage 10 est intégré au sein de l'ensemble 1 de la figure 1, la deuxième pièce 42B est fixée au support 6 de la béquille 4, par exemple au moyen de vis (non représentées). La deuxième pièce 42B est visible en détails sur les figures 11 et 16. Selon cet exemple de réalisation, la deuxième pièce 42B est de forme par exemple sensiblement plane et évidée en son centre 58, pour permettre le passage de l'axe 40. Le dispositif de verrouillage 10 comporte en outre un système de butées 60, 62 entre la première pièce 42A et la deuxième pièce 42B, de sorte à contraindre la première pièce 42A dans son débattement angulaire. Plus précisément, selon un exemple de réalisation particulier représenté sur les figures 11 et 16, la deuxième pièce 42B est munie d'un ergot longitudinal 60 qui s'étend parallèlement à la direction longitudinale D2, depuis une surface de la deuxième pièce 42B opposée à la surface de fixation au support 6 de béquille. Selon cet exemple de réalisation particulier, la première pièce 42A est munie sur son pourtour extérieur d'un ergot transversal 62 en butée contre l'ergot longitudinal 60 de la deuxième pièce 42B. Un tel ergot transversal 62 est visible aux figures 11 et 14. Par conséquent, lorsqu'un utilisateur fait pivoter la poignée 2 dans le sens horaire S3, la poignée 2 fixée à la deuxième pièce 42B par l'intermédiaire du support 6 fait pivoter la première pièce 42A en sens horaire S3, au moyen de l'ergot longitudinal 60 qui agit sur l'ergot transversal 62. La première pièce 42A fait alors pivoter l'axe 40 au moyen des butées 54 qui glissent dans les fentes 50 et coopèrent avec un bord intérieur 56 de l'axe 40, entraînant la rotation de l'axe 40 dans le sens horaire S3. L'axe 40 entraine alors avec lui le carré de pêne demi-tour de la porte, provoquant le retrait du pêne demi-tour de son logement dans la porte, et donc l'ouverture de celle-ci.

Une troisième pièce 42C forme une troisième liaison pivot 52C avec l'axe 40. La troisième pièce 42C est agencée entre les première et deuxième pièces 42A, 42B, et est en outre liée mécaniquement au système de transformation de mouvement pivot 44. La troisième pièce 42C est visible en détails sur les figures 11, 12 et 15. Le dispositif de verrouillage 10 comporte en outre un système de butées 60, 64, 66, 68A, 68B entre la troisième pièce 42C d'une part et la première pièce 42A ou la deuxième pièce 42B d'autre part. Plus précisément, selon l'exemple de réalisation des figures 11, 12 et 15, la troisième pièce 42C est évidée en son centre 70, pour permettre le passage de l'axe 40. La troisième pièce 42C est munie d'une portion périphérique dentée 72 en prise avec le système de transformation de mouvement pivot 44, d'une portion périphérique formant butée support 64, et d'une portion périphérique 66 présentant une échancrure 78 pour permettre le passage de l'ergot longitudinal 60 de la deuxième pièce 42B. La portion périphérique formant butée support 64 est plus épaisse que deux autres portions périphériques 72, 66, l'épaisseur étant considérée comme la dimension qui s'étend selon la direction longitudinale D2. Selon cet exemple de réalisation particulier, la première pièce 42A est munie sur son pourtour extérieur de deux ergots transversaux 68A, 68B. Ces deux ergots transversaux 68A, 68B sont visibles aux figures 12 et 14. Un premier ergot transversal 68A est en butée contre la portion périphérique formant butée support 64, et forme une butée d'arrêt de la troisième pièce 42C par rapport à la première pièce 42A. Un deuxième ergot transversal 68B est disposé de l'autre côté de la portion périphérique formant butée support 64, par rapport au premier ergot transversal 68A, et est apte à appuyer sur cette portion périphérique 64 lors d'une rotation de la première pièce 42A dans le sens horaire S3, et à entraîner ainsi la troisième pièce 42C en rotation dans le sens S3.

L'ergot longitudinal 60 est en butée contre la portion périphérique 66, comme illustré sur la figure 11. De cette façon, un mouvement de rotation de la deuxième pièce 42B selon le sens anti-horaire S4 entraine une rotation de la troisième pièce 42C selon le même sens de rotation S4, et donc, via l'action de la portion périphérique dentée 72, un mouvement du système de transformation de mouvement pivot 44, pour le verrouillage de la porte. Plus précisément, et comme illustré sur les figures 10 et 11, lorsqu'un utilisateur fait pivoter la poignée 2 dans le sens anti-horaire S4, la poignée 2 fixée à la deuxième pièce 42B par l'intermédiaire du support 6 fait pivoter la troisième pièce 42C en sens anti-horaire S4, au moyen de l'ergot longitudinal 60 qui agit sur la portion périphérique 66. La troisième pièce 42C entraine alors, via la portion périphérique dentée 72, un mouvement du système de transformation de mouvement pivot 44, qui agit sur le carré de pêne dormant pour le verrouillage de la porte. Une fois en position de verrouillage du dispositif 10 et donc de la porte, l'utilisateur peut ensuite déverrouiller celle-ci par pivotement de la poignée 2 dans le sens horaire S3. Plus précisément, en position de verrouillage du dispositif 10, lorsque l'utilisateur fait pivoter la poignée 2 dans le sens horaire S3, la poignée 2 fixée à la deuxième pièce 42B par l'intermédiaire du support 6 fait pivoter la première pièce 42A en sens horaire S3, au moyen de l'ergot longitudinal 60 qui agit sur l'ergot transversal 62. Ceci conduit au retrait du pêne demi-tour de son logement, via la rotation de l'axe 40, comme indiqué précédemment. En outre, et comme illustré sur la figure 12, cette rotation de la première pièce 42A en sens horaire S3 entraine une rotation de la troisième pièce 42C selon le même sens S3, via l'action du deuxième ergot transversal 68B sur la portion périphérique formant butée support 64. La troisième pièce 42C entraine alors, via la portion périphérique dentée 72, un mouvement du système de transformation de mouvement pivot 44, qui agit sur le carré de pêne dormant pour le déverrouillage de la porte. La troisième pièce 42C revient alors à sa position initiale, correspondant à une position de déverrouillage de la porte.

Comme illustré sur les figures 9 et 10, le système de transformation de mouvement pivot 44 est par exemple un ensemble à engrenages, sans que cela ne soit limitatif dans le cadre de la présente invention. Le système de transformation de mouvement pivot 44 comprend par exemple une première roue dentée 80 en prise avec la portion périphérique dentée 72 de la troisième pièce 42C, et une seconde roue dentée 82 solidaire en rotation du carré de pêne dormant de la porte. Le carré de pêne dormant de la porte agit sur le verrouillage ou le déverrouillage de cette dernière. Le système de transformation de mouvement pivot 44 est par exemple configuré pour transformer, via les roues dentées 80, 82, un mouvement angulaire de 45 degrés sur la troisième pièce 42C en un mouvement angulaire correspondant de 90 degrés sur le carré de pêne dormant.

## Revendications

1. Système (8) de nettoyage et/ou de désinfection d'un tube creux (4), comprenant :
- le tube creux (4) ;
- une bague (18) montée coulissante sur le tube creux (4), la bague (18) renfermant un tampon absorbant imbibé d'un liquide désinfectant et/ou dégraissant, le tampon absorbant étant agencé autour du tube creux (4) ;
- un dispositif (20) d'actionnement électromécanique de la bague (18) agencé à l'intérieur du tube creux (4), ledit dispositif (20) comportant un élément (26) d'entrainement en translation longitudinale ;
- une fente longitudinale (11) est ménagée dans le tube creux (4), l'élément (26) d'entrainement en translation longitudinale faisant saillie à travers ladite fente longitudinale (11) et étant mécaniquement lié à la bague (18), pour le déplacement de la bague (18) en translation longitudinale le long du tube creux (4) ;
**caractérisé en ce que** le dispositif d'actionnement électromécanique (20) est un potentiomètre linéaire motorisé.

2. Système (8) selon la revendication 1, **caractérisé en ce que** le tube creux (4) est une béquille de manoeuvre d'une poignée de porte (2).

3. Système (8) selon la revendication 1 ou 2, **caractérisé en ce que** le dispositif d'actionnement électromécanique (20) comporte en outre un moteur (28) alimenté électriquement et un ensemble poulies/courroies (30) actionné par le moteur (28), l'élément (26) d'entrainement en translation longitudinale étant relié à l'ensemble poulies/courroies (30).

4. Système (8) selon l'une quelconque des revendications précédentes, **caractérisé en ce que** l'élément (26) d'entrainement en translation longitudinale est un chariot mobile monté en translation sur une partie fixe (36) du potentiomètre linéaire motorisé et comportant une languette (34), et **en ce qu'**une fente longitudinale (23) est ménagée dans la bague (18), la languette (34) faisant saillie à travers les fentes longitudinales respectives (11, 23) du tube creux (4) et de la bague (18).

5. Système (8) selon l'une quelconque des revendications précédentes, **caractérisé en ce que** le tube creux (4) ou la bague (18) est muni(e) d'au moins un marquage visuel, notamment au moins un marquage coloré ou au moins un repère gravé, ledit au moins un marquage visuel étant indicatif d'une rupture en liquide et/ou en alimentation électrique.

6. Système (8) selon l'une quelconque des revendications précédentes lorsqu'elle dépend de la revendication 3, **caractérisé en ce que** le dispositif d'actionnement électromécanique (20) comporte en outre un microcontrôleur de commande du moteur (28), relié au moteur (28).

7. Système (8) selon la revendication 6 lorsqu'elle dépend de la revendication 2, **caractérisé en ce que** le dispositif d'actionnement électromécanique (20) comporte en outre un inclinomètre relié au microcontrôleur, ledit inclinomètre étant configuré pour détecter un mouvement de la béquille (4) et pour transmettre au microcontrôleur un signal de détection correspondant, le microcontrôleur étant configuré pour transmettre au moteur (28), à l'issue d'une durée prédéfinie écoulée depuis la réception du signal de détection, un signal de commande d'un mouvement de va-et-vient de la bague (18) le long de la béquille (4).

8. Système (8) selon la revendication 6 ou 7, **caractérisé en ce que** le microcontrôleur comprend des moyens de traitement et des moyens mémoire stockant un code source, le code source comportant des instructions de programme qui, lorsqu'exécutées par les moyens de traitement, déclenchent une séquence de commande du moteur (28), pour le déplacement de la bague (18) en translation longitudinale le long du tube creux (4).

9. Système (8) selon la revendication 8 lorsqu'elle dépend de la revendication 5, **caractérisé en ce que** le microcontrôleur comprend en outre un compteur pré-étalonné, le compteur enregistrant un nombre prédéterminé de cycles du système (8), correspondant à une rupture en liquide et/ou en alimentation électrique, et **en ce que** la séquence de commande du moteur (28) est paramétrée au sein du code source de sorte que, lorsque le compteur a atteint le nombre prédéterminé de cycles, le microcontrôleur commande le moteur (28) pour amener la bague (18), en fin de mouvement, jusqu'à une position prédéfinie sur le tube creux (4) laissant apparaître le marquage visuel.

10. Système (8) selon l'une quelconque des revendications précédentes, **caractérisé en ce qu'**il comprend en outre un réservoir (16) de liquide désinfectant et/ou dégraissant, la bague (18) étant solidarisée au réservoir (16), le tampon absorbant étant en communication fluidique avec le réservoir (16).

11. Système (8) selon la revendication 10 lorsqu'elle dépend de la revendication 2, **caractérisé en ce que** la bague (18) et le réservoir (16) forment une pièce monobloc, et **en ce qu'**un premier orifice (22A) est ménagé dans la bague (18) en regard du tampon absorbant, ledit orifice (22A) débouchant dans le réservoir (16), le réservoir (16) et l'orifice (22A) étant configurés pour que du liquide s'écoule depuis le réservoir (16) vers le tampon, à travers l'orifice (22A), lorsque la béquille (4) est inclinée vers le bas.

12. Système (8) selon la revendication 10 ou 11, **caractérisé en ce que** la bague (18) et le réservoir (16) forment une pièce monobloc, **en ce qu'**un second orifice (22B) est ménagé dans la bague (18) en regard du tampon absorbant, ledit orifice (22B) débouchant dans le réservoir (16), et **en ce que** le système (8) comporte en outre une mèche capillaire agencée à travers l'orifice (22B) jusque dans le réservoir (16), et en contact avec le tampon absorbant, pour permettre un transfert de liquide par capillarité du réservoir (16) vers le tampon absorbant.

13. Système (8) selon la revendication 1 ou l'une des revendications 3 à 6, 8 à 10, et 12, **caractérisé en ce que** le tube creux (4) est un tube d'une barre équipant un moyen de transport, d'une poignée de type bâton de maréchal ou d'une rampe telle que notamment une rampe d'escalier.

14. Ensemble (1) comprenant au moins une poignée de porte (2) munie d'une béquille (4) de manoeuvre de la poignée (2) et d'un support (6) de la béquille (4), l'ensemble (1) comprenant en outre au moins un système (8) de nettoyage et/ou de désinfection d'un tube creux (4), **caractérisé en ce que** le système de nettoyage et/ou de désinfection (8) est conforme à l'une quelconque des revendications précédentes, le tube creux (4) constituant la béquille.

15. Ensemble (1) selon la revendication 14, **caractérisé en ce qu'**il comprend en outre un dispositif (10) de verrouillage de la porte par action sur la béquille (4), ledit dispositif (10) comportant un axe (40) solidaire en rotation d'un carré de pêne demi-tour de la porte, et trois pièces (42A, 42B, 42C) montées autour de l'axe (40) et formant chacune une liaison pivot (52A, 52B, 52C) avec l'axe (40), une première pièce (42A) formant une première liaison pivot (52A) avec l'axe (40) ; une deuxième pièce (42B) étant fixée au support (6) de la béquille (4) et formant une deuxième liaison pivot (52B) avec l'axe (40), et une troisième pièce (42C) agencée entre les première et deuxième pièces (42A, 42B) et formant une troisième liaison pivot (52C) avec l'axe (40) ; le dispositif (10) comportant en outre un premier système de butées (60, 62) entre la première pièce (42A) et la deuxième pièce (42B), de sorte à contraindre la première pièce (42A) dans son débattement angulaire ; et un second système de butées (60, 64, 66, 68B) entre la troisième pièce (42C) d'une part et la première pièce (42A) ou la deuxième pièce (42B) d'autre part ; le dispositif de verrouillage (10) comportant en outre un système (44) de transformation du mouvement pivot de la troisième pièce (42C) en un mouvement pivot sur un carré de pêne dormant de la porte, pour le verrouillage ou le déverrouillage de la porte.

16. Ensemble (1) selon la revendication 15, **caractérisé en ce que** la troisième pièce (42C) et le système de transformation de mouvement pivot (44) sont configurés de sorte qu'un mouvement de la deuxième pièce (42B) dans un premier sens de rotation (S4) entraîne un mouvement de la troisième pièce (42C) et du système de transformation de mouvement pivot (44), pour le verrouillage de la porte, et qu'un mouvement de la deuxième pièce (42B) dans un second sens de rotation (S3), opposé au premier sens (S4), entraîne un mouvement de la première pièce (42A) et de l'axe (40), pour l'ouverture de la porte.

17. Ensemble (1) selon la revendication 15 ou 16, **caractérisé en ce que** la première liaison pivot (52A) est configurée pour être sur le débattement angulaire de la poignée du côté de la porte opposé au côté d'installation du dispositif de verrouillage (10), de sorte qu'une inclinaison de la béquille (4) du côté de la porte opposé au côté d'installation du dispositif (10), entraînant une rotation de l'axe (40), est découplée de tout mouvement sur la première pièce (42A).

## Patentansprüche

1. System (8) zum Reinigen und/oder Desinfizieren eines Hohlrohrs (4), umfassend:
- das Hohlrohr (4);
- einen Ring (18), der verschiebbar auf dem Hohlrohr (4) angebracht ist, wobei der Ring (18) einen absorbierenden Bausch einschließt, der mit einer desinfizierenden und/oder entfettenden Flüssigkeit getränkt ist, wobei der absorbierende Bausch um das Hohlrohr (4) herum angeordnet ist;
- eine Vorrichtung (20) zum elektromechanischen Antrieb des Rings (18), die innerhalb des Hohlrohrs (4) angeordnet ist, wobei die Vorrichtung (20) ein längsverschiebbares Führungselement (26) aufweist;
- eine Längsspalte (11), die in dem Hohlrohr (4) vorgesehen ist, wobei das längsverschiebbare Führungselement (26) durch die Längsspalte (11) vorsteht und mit dem Ring (18) für die Versetzung des längsverschiebbaren Rings (18) entlang des Hohlrohrs (4) mechanisch verbunden ist;
**dadurch gekennzeichnet, dass** die Vorrichtung (20) zum elektromechanischen Antrieb ein motorisiertes lineares Potentiometer ist.

2. System (8) nach Anspruch 1, **dadurch gekennzeichnet, dass** das Hohlrohr (4) eine Klinke zum Betätigen eines Türgriffs (2) ist.

3. System (8) nach Anspruch 1 oder 2, **dadurch gekennzeichnet, dass** die Vorrichtung (20) zum elektromechanischen Antrieb ferner einen elektrisch gespeisten Motor (28) und eine durch den Motor (28) angetriebene Riemenantriebsanordnung (30) aufweist, wobei das längsverschiebbare Führungselement (26) mit der Riemenantriebsanordnung (30) verbunden ist.

4. System (8) nach einem der vorstehenden Ansprüche, **dadurch gekennzeichnet, dass** das längsverschiebbare Führungselement (26) ein mobiler Schlitten ist, der auf einem festen Teil (36) des motorisierten linearen Potentiometers angebracht ist und eine Zunge (34) aufweist, und dass in dem Ring (18) eine Längsspalte (23) vorgesehen ist, wobei die Zunge (34) durch die jeweiligen Längsspalten (11, 23) des Hohlrohrs (4) und des Rings (18) vorsteht.

5. System (8) nach einem der vorstehenden Ansprüche, **dadurch gekennzeichnet, dass** das Hohlrohr (4) oder der Ring (18) mit mindestens einer visuellen Markierung, insbesondere mindestens einer farbigen Markierung oder mindestens einem geprägten Zeichen, versehen ist, wobei die mindestens eine visuelle Markierung eine Unterbrechung der Flüssigkeit und/oder der elektrischen Speisung anzeigt.

6. System (8) nach einem der vorstehenden Ansprüche, wenn abhängig von Anspruch 3, **dadurch gekennzeichnet, dass** die Vorrichtung (20) zum elektromechanischen Antrieb ferner einen Mikrocontroller einer Steuerung des Motors (28) umfasst, der mit dem Motor (28) verbunden ist.

7. System (8) nach Anspruch 6, wenn abhängig von Anspruch 2, **dadurch gekennzeichnet, dass** die Vorrichtung (20) zum elektromechanischen Antrieb ferner einen Neigungsmesser aufweist, der mit dem Mikrocontroller verbunden ist, wobei der Neigungsmesser konfiguriert ist, um eine Bewegung der Klinke (4) zu erfassen und um ein entsprechendes Erfassungssignal an den Mikrocontroller zu übertragen, wobei der Mikrocontroller konfiguriert ist, um nach einer vordefinierten Zeitspanne, die seit dem Empfang des Erfassungssignals vergangen ist, ein Steuersignal für eine hin- und hergehende Bewegung des Rings (18) entlang der Klinke (4) an den Motor (28) zu übertragen.

8. System (8) nach Anspruch 6 oder 7, **dadurch gekennzeichnet, dass** der Mikrocontroller Verarbeitungsmittel und Speichermittel umfasst, die einen Quellcode speichern, wobei der Quellcode Programmanweisungen aufweist, die, wenn sie durch die Verarbeitungsmittel ausgeführt werden, eine Steuersequenz des Motors (28) für die Versetzung des längsverschiebbaren Rings (18) entlang des Hohlrohrs (4) auslösen.

9. System (8) nach Anspruch 8, wenn abhängig von Anspruch 5, **dadurch gekennzeichnet, dass** der Mikrocontroller ferner einen vorkalibrierten Zähler umfasst, wobei der Zähler eine vorbestimmte Anzahl von Zyklen des Systems (8) registriert, entsprechend einer Unterbrechung der Flüssigkeit und/oder der elektrischen Speisung, und dass die Steuersequenz des Motors (28) innerhalb des Quellcodes parametriert ist, sodass, wenn der Zähler die vorbestimmte Anzahl von Zyklen erreicht hat, der Mikrocontroller den Motor (28) steuert, um den Ring (18) an einem Ende der Bewegung zu einer vordefinierten Position auf dem Hohlrohr (4) mitzunehmen, wobei die visuelle Markierung erkannt werden kann.

10. System (8) nach einem der vorstehenden Ansprüche, **dadurch gekennzeichnet, dass** es ferner einen Behälter (16) für desinfizierende und/oder entfettende Flüssigkeit umfasst, wobei der Ring (18) an dem Behälter (16) befestigt ist, wobei der absorbierende Bausch mit dem Behälter (16) in Fluidverbindung steht.

11. System (8) nach Anspruch 10, wenn abhängig von Anspruch 2, **dadurch gekennzeichnet, dass** der Ring (18) und der Behälter (16) ein einstückiges Teil ausbilden und dass eine erste Öffnung (22A) in dem Ring (18) gegenüber dem absorbierenden Bausch vorgesehen ist, wobei die Öffnung (22A) in den Behälter (16) mündet, wobei der Behälter (16) und die Öffnung (22A) konfiguriert sind, um Flüssigkeit aus dem Behälter (16) in Richtung des Bausches durch die Öffnung (22A) auslaufen zu lassen, wenn die Klinke (4) nach unten geneigt ist.

12. System (8) nach Anspruch 10 oder 11, **dadurch gekennzeichnet, dass** der Ring (18) und der Behälter (16) ein einstückiges Teil ausbilden, dass eine zweite Öffnung (22B) in dem Ring (18) gegenüber dem absorbierenden Bausch vorgesehen ist, wobei die Öffnung (22B) in den Behälter (16) mündet, und dass das System (8) ferner eine Kapillarbohrung aufweist, die durch die Öffnung (22B) bis in den Behälter (16) angeordnet ist, und mit dem absorbierenden Bausch in Kontakt ist, um eine Übertragung von Flüssigkeit durch Kapillarwirkung von dem Behälter (16) zu dem absorbierenden Bausch zu ermöglichen.

13. System (8) nach Anspruch 1 oder einem der Ansprüche 3 bis 6, 8 bis 10 und 12, **dadurch gekennzeichnet, dass** das Hohlrohr (4) ein Rohr einer Stange, die ein Transportmittel ausstattet, eines Griffs einer Art eines Marschallstabs oder eines Geländers, wie insbesondere eines Treppengeländers, ist.

14. Anordnung (1), umfassend mindestens einen Türgriff (2), der mit einer Klinke (4) zum Betätigen des Griffs (2) und einer Halterung (6) der Klinke (4) versehen ist, die Anordnung (1) ferner umfassend mindestens ein System (8) zum Reinigen und/oder Desinfizieren eines Hohlrohrs (4), **dadurch gekennzeichnet, dass** das System (8) zum Reinigen und/oder Desinfizieren mit einem der vorstehenden Ansprüche übereinstimmt, wobei das Hohlrohr (4) die Klinke ausmacht.

15. Anordnung (1) nach Anspruch 14, **dadurch gekennzeichnet, dass** sie ferner eine Vorrichtung (10) zum Verriegeln der Tür durch Einwirkung auf die Klinke (4), wobei die Vorrichtung (10) einen Zapfen (40) aufweist, der mit dem Klinkenvierkant der Tür drehfest verbunden ist, und drei Teile (42A, 42B, 42C) umfasst, die um den Zapfen (40) herum angebracht sind und jeweils eine Schwenkverbindung (52A, 52B, 52C) mit dem Zapfen (40) ausbilden, wobei ein erstes Teil (42A) eine erste Schwenkverbindung (52A) mit dem Zapfen (40) ausbildet; ein zweites Teil (42B) an der Halterung (6) der Klinke (4) befestigt ist und eine zweite Schwenkverbindung (52B) mit dem Zapfen (40) ausbildet, und ein drittes Teil (42C) zwischen dem ersten und dem zweiten Teil (42A, 42B) angeordnet ist und eine dritte Schwenkverbindung (52C) mit der Achse (40) ausbildet; wobei die Vorrichtung (10) ferner ein erstes Anschlagssystem (60, 62) zwischen dem ersten Teil (42A) und dem zweiten Teil (42B), um das erste Teil (42A) in seine Winkelauslenkung zu zwingen; und ein zweites Anschlagssystem (60, 64, 66, 68B) zwischen dem dritten Teil (42C) einerseits und dem ersten Teil (42A) oder dem zweiten Teil (42B) andererseits aufweist; wobei die Verriegelungsvorrichtung (10) ferner ein System (44) zum Umwandeln der Schwenkbewegung des dritten Teils (42C) in eine Schwenkbewegung an einem Schlossriegelvierkant der Tür für das Verriegeln oder das Entriegeln der Tür aufweist.

16. Anordnung (1) nach Anspruch 15, **dadurch gekennzeichnet, dass** das dritte Teil (42C) und das System (44) zum Umwandeln der Schwenkbewegung konfiguriert sind, sodass eine Bewegung des zweiten Teils (42B) in einer ersten Drehrichtung (S4) eine Bewegung des dritten Teils (42C) und des Systems (44) zum Umwandeln der Schwenkbewegung, zum Verriegeln der Tür, herbeiführt, und dass eine Bewegung des zweiten Teils (42B) in einer zweiten Drehrichtung (S3), entgegengesetzt zu der ersten Richtung (S4), eine Bewegung des ersten Teils (42A) und des Zapfens (40), zum Öffnen der Tür, herbeiführt.

17. Anordnung (1) nach Anspruch 15 oder 16, **dadurch gekennzeichnet, dass** die erste Schwenkverbindung (52A) konfiguriert ist, um auf der Winkelauslenkung des Griffs auf der Seite der Tür zu sein, die der Installationsseite der Verriegelungsvorrichtung (10) gegenüberliegt, sodass eine Neigung der Klinke (4) auf der Seite der Tür, die der Installationsseite der Vorrichtung (10) gegenüberliegt, die eine Drehung des Zapfens (40) herbeiführt, von jeder Bewegung auf dem ersten Teil (42A) entkoppelt wird.

## Claims

1. System (8) for cleaning and/or disinfecting a hollow tube (4), comprising:
- the hollow tube (4);
- a ring (18) slidably mounted on the hollow tube (4), the ring (18) containing an absorbent pad soaked with a disinfecting and/or degreasing liquid, the absorbent pad being arranged around the hollow tube (4);
- a device (20) for electromechanical actuation of the ring (18), which is arranged inside the hollow tube (4), said device (20) comprising an element (26) for driving in longitudinal translation;
- a longitudinal slot (11) is provided in the hollow tube (4), the element (26) for driving in longitudinal translation protruding through said longitudinal slot (11) and being mechanically connected to the ring (18), in order to move the ring (18) in longitudinal translation along the hollow tube (4);
**characterized in that** the electromechanical actuation device (20) is a motor-driven linear potentiometer.

2. System (8) according to claim 1, **characterized in that** the hollow tube (4) is a lever for operating a door handle (2).

3. System (8) according to claim 1 or 2, **characterized in that** the electromechanical actuation device (20) further comprises an electrically powered motor (28) and a pulley/belt assembly (30) actuated by the motor (28), the element (26) for driving in longitudinal translation being connected to the pulley/belt assembly (30).

4. System (8) according to any of the preceding claims, **characterized in that** the element (26) for driving in longitudinal translation is a movable carriage translatably which is mounted on a fixed portion (36) of the motor-driven linear potentiometer and comprises a tab (34), and **in that** a longitudinal slot (23) is provided in the ring (18), the tab (34) projecting through the respective longitudinal slots (11, 23) of the hollow tube (4) and of the ring (18).

5. System (8) according to any of the preceding claims, **characterized in that** the hollow tube (4) or the ring (18) are provided with at least one visual marking, in particular at least one colored marking or at least one etched marking, said at least one visual marking being indicative of a halt in the supply of liquid and/or electrical power.

6. System (8) according to any of the preceding claims when dependent on claim 3, **characterized in that** the electromechanical actuation device (20) further comprises a microcontroller (28) for controlling the motor, connected to the motor (28).

7. System (8) according to claim 6 when dependent on claim 2, **characterized in that** the electromechanical actuation device (20) further comprises an inclinometer connected to the microcontroller, said inclinometer being configured to detect a movement of the lever (4) and to transmit a corresponding detection signal to the microcontroller, the microcontroller being configured to transmit to the motor (28), at the end of a predefined period of time following the reception of the detection signal, a control signal for a reciprocating movement of the ring (18) along the lever (4).

8. System (8) according to claim 6 or 7, **characterized in that** the microcontroller comprises processing means and memory means storing a source code, the source code comprising program instructions which, when executed by the processing means, trigger a sequence for controlling the motor (28) in order to move the ring (18) in longitudinal translation along the hollow tube (4).

9. System (8) according to claim 8 when dependent on claim 5, **characterized in that** the microcontroller further comprises a pre-calibrated counter, the counter recording a predetermined number of cycles of the system (8), corresponding to a halt in the supply of liquid and/or electrical power, and **in that** the control sequence for the motor (28) is parameterized within the source code so that, when the counter has reached the predetermined number of cycles, the microcontroller controls the motor (28) to bring the ring (18), at the end of movement, to a predetermined position on the hollow tube (4) thus revealing the visual marking.

10. System (8) according to any of the preceding claims, **characterized in that** it further comprises a container (16) for disinfecting and/or degreasing liquid, the ring (18) being secured to the container (16), and the absorbent pad being in fluid communication with the container (16).

11. System (8) according to claim 10 when dependent on claim 2, **characterized in that** the ring (18) and the container (16) form a single piece, and **in that** a first opening (22A) is provided in the ring (18) facing the absorbent pad, said opening (22A) leading into the container (16), the container (16) and the opening (22A) being configured so that liquid flows from the container (16) towards the pad through the opening (22A) when the lever (4) is tilted downwards.

12. System (8) according to claim 10 or 11, **characterized in that** the ring (18) and the container (16) form a single piece, **in that** a second opening (22B) is provided in the ring (18) facing the absorbent pad, said opening (22B) leading into the container (16), and **in that** the system (8) further comprises a capillary wick arranged through the opening (22B) into the container (16), and in contact with the absorbent pad, thus allowing liquid transfer by capillary action from the container (16) to the absorbent pad.

13. System (8) according to claim 1 or any of claims 3 to 6, 8 to 10, and 12, **characterized in that** the hollow tube (4) is a tube of a bar provided in a means of transport, of a handle in the style of a field marshal's baton, or of a handrail such as in particular a staircase handrail.

14. Assembly (1) comprising at least one door handle (2) provided with a lever (4) for operating the handle (2) and a support member (6) for the lever (4), the assembly (1) further comprising at least one system (8) for cleaning and/or disinfecting a hollow tube (4), **characterized in that** the cleaning and/or disinfecting system (8) conforms to any of the preceding claims, the hollow tube (4) constituting the lever.

15. Assembly (1) according to claim 14, **characterized in that** it further comprises a device (10) for locking the door by acting on the lever (4), said device (10) comprising a shaft (40) constrained to rotate with a square latch bolt of the door, and three parts (42A, 42B, 42C) mounted around the shaft (40) and each forming a pivot connection (52A, 52B, 52C) with the shaft (40), a first part (42A) forming a first pivot connection (52A) with the shaft (40); a second part (42B) being secured to the support member (6) for the lever (4) and forming a second pivot connection (52B) with the shaft (40), and a third part (42C) arranged between the first and second parts (42A, 42B) and forming a third pivot connection (52C) with the shaft (40); the device (10) further comprising a first system of stops (60, 62) between the first part (42A) and the second part (42B), so as to force the first part (42A) into its angular range of movement; and a second system of stops (60, 64, 66, 68B) between the third part (42C) on the one hand, and the first part (42A) or the second part (42B) on the other hand; the locking device (10) further comprising a system (44) for converting the pivot movement of the third part (42C) into a pivot movement of a square deadbolt of the door, for locking or unlocking the door.

16. Assembly (1) according to claim 15, **characterized in that** the third part (42C) and the system (44) for converting the pivot movement are configured so that a movement of the second part (42B) in a first direction of rotation (S4) causes a movement of the third part (42C) and the system (44) for converting the pivot movement, for locking the door, and **in that** a movement of the second part (42B) in a second direction of rotation (S3), opposite to the first direction of rotation (S4), causes a movement of the first part (42A) and the shaft (40), for opening the door.

17. Assembly (1) according to claim 15 or 16, **characterized in that** the first pivot connection (52A) is configured to be on the angular range of movement of the handle on the side of the door opposite to the side of installation of the locking device (10), so that a tilting of the lever (4) on the side of the door opposite to the side of installation of locking device (10), causing a rotation of the shaft (40), is decoupled from any movement on the first part (42A).
